# EUROPEAN PATENT APPLICATION

(11) **EP 1 857 044 A2**
(43) Date of publication of application: **21.11.2007**
(21) Application number: 07251264.3
(22) Date of filing: 24.03.2007
(51) Int. Cl.: A61B 3/135

(54) **Flash generation and synchronisation for slit lamp microscope**

(30) Priority: 16.05.2006 GB 0609658
(71) Applicant: Litechnica Ltd., Kirby House, 122 Heston Road Heston Middlesex, TW5 0QU (GB)
(72) Inventor: Gutridge, John Robert, Hampton Middlesex, TW12 2TH (GB); Snellgrove, Timothy Ralph, Marple Stockport, SK6 7HJ (GB)
(74) Representative: Lawrence, John

(57) **Abstract**

A bulb assembly 30 for a slit lamp microscope comprises flash generation means 33 able to produce a flash of light, synchronisation means 41 able to detect light from a camera flash, and a controller 32. The bulb assembly 30 is arranged so that, in use, when the controller receives a signal from the synchronisation means 41 indicating light from a camera flash has been detected, the controller causes the flash generation means 33 to produce a slave flash of light. The bulb assembly may further comprise a light source 31 and light from the light source may be focussed onto the flash generation means 33 so as to produce a virtual image 37 of the light source 31 in the same physical location as the flash generation means.

## Description

The present invention relates to a slit lamp microscope. More particularly, but not exclusively, the invention relates to a bulb assembly for a slit lamp microscope, arranged to allow the microscope, in conjunction with a camera, to be used a take a photograph of the interior of a patient's eye.

A slit lamp microscope is a diagnostic tool commonly used by ophthalmologists and surgeons to examine a patient's eye. A patient is positioned in front of the microscope, and a line of light is shone into the patient's eye. The ophthalmologist can then examine the illuminated retina of the patient through the eyepiece of the slit lamp microscope.

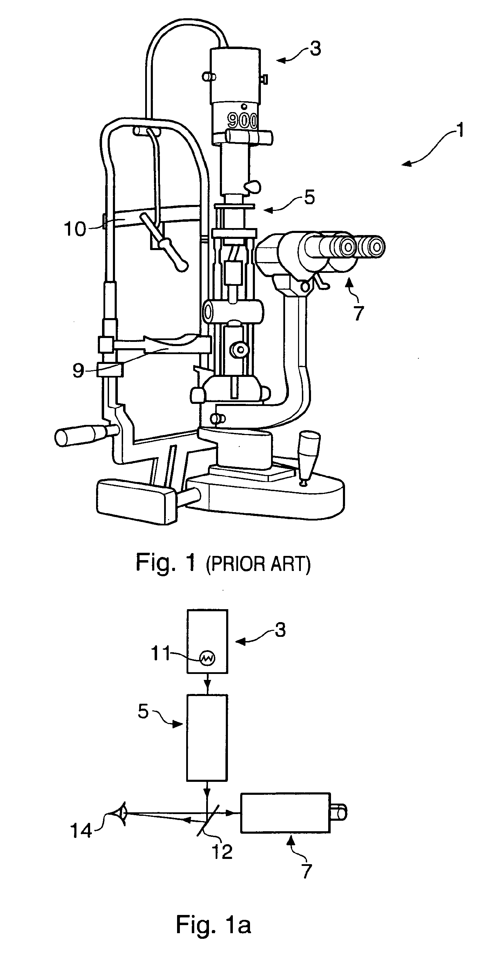

The line of light can be moved and focussed by the ophthalmologist in order to direct it towards an area of particular interest within the eye. A commonly used slit lamp microscope is the Haag Streit BM900, which allows the beam of light to be tilted and rotated by the user. The Haag Streit BM900 is shown in Figure 1 (prior art).

Often an ophthalmologist or clinician may wish to take a photograph of the retina of a patient's eye. There are many reasons why this might be desirable. For example, a photograph would allow the clinician to obtain a second opinion on diagnosis from another clinician (or multiple clinicians) who is (are) not present. A photograph could be taken and emailed to a specialist for remote diagnoses. At present it is common for ophthalmologists to draw pictures of the interior of the eye for these purposes, which can be time consuming and inaccurate. The photograph could be used as a learning aid for trainees, or could be used to explain to the patient himself a problem with his retina. A photographic record of how a patient's retina has changed (if at all) over time would be useful in order to monitor the progress of a condition.

There are two main ways in which a photograph of a patient's retina can be obtained using a slit lamp microscope. The first, and most prevalent, way is to attach a digital camera to the eyepiece of a standard slit lamp microscope, such as the BM900. However, we have found this to produce unsatisfactory pictures, because of the low level of illumination available.

A slit lamp microscope directs only a small amount of light into a patient's eye. This allows a clinician to view a patient's retina without causing excessive discomfort to the patient by prolonged exposure to bright light. Thus only a very low level of light is directed onto the subject of the photograph. In order to produce a picture that is not overly dark, it is necessary to use a long shutter time, which results in poor quality pictures. Long exposures introduce unwanted noise into the resulting image, and the resulting picture may be blurred due to the patient moving during the exposure. The camera's own flash is of no assistance in this situation, as it is not directed at the patient's retina through the optical system of the slit lamp.

A solution to this problem is provided by the Haag Streit BX900 slit lamp. This slit lamp microscope comprises an integrated camera mounted above the eyepiece of the microscope, and an integrated flash unit. A mirror housing is provided to allow light from the retina to be directed either towards the eyepiece of the microscope or towards the camera. While the BX900 produces good pictures of the retina, it is relatively expensive (perhaps of the order of £20,000). If an ophthalmologist already has a standard slit lamp microscope, it is expensive for him to buy a completely new slit lamp microscope solely in order to take photographs of the eye. This additional microscope may take up much needed space in a surgery, and may stand unused for a large portion of the ophthalmologist's normal working day.

Thus, at the priority date of this application, if a photograph of a patient's retina is wanted, it is necessary for an ophthalmologist to choose between purchasing a second expensive piece of equipment, and making do with poor quality pictures.

According to a first aspect of the invention we provide a slit lamp microscope comprising an optical axis, flash generation means able to generate a flash of light, synchronisation means arranged to detect light from a camera flash, and a flash controller, the microscope being arranged so that, in use, when the flash controller receives a signal from the synchronisation means indicating that light from a camera flash has been detected, the controller causes the flash generation means to produce a slave flash of light, such that at least some of the light from the slave flash travels along the optical axis of the microscope.

Light from the slave flash thus illuminates a subject on the optical axis of the microscope temporarily a negligible time after the camera flash is detected, synchronising the slave flash with the opening of the camera's shutter.

Preferably the flash generation means, synchronisation means and controller are part of a self-contained bulb assembly.

Preferably, the flash generation means is substantially on the optical axis of the microscope.

When the microscope user has his desired subject visible in an eyepiece, he can then position a camera adjacent the eyepiece of the microscope (if the camera is not already mounted at the or an eye piece), and operate the camera's shutter release mechanism. The flash generation means of the bulb assembly produces a slave flash a negligible time after the camera's own flash fires, illuminating the subject being viewed as the camera's shutter opens, resulting in a photograph being taken of an area of the interior of the eye having a relatively high level of illumination by a standard, camera, which is not integral with the ophthalmoscope. The camera may be a photographic film or digital camera, but is preferably a digital camera.

Modern digital cameras use two flashes: an initial test of sampling flash, and a main flash, which fire in quick succession. The flash generation means should not generate a flash before the camera's shutter opens, or the flash will have died away by the time the picture is taken, resulting in a picture that is too dark. Preferably the flash generation means is arranged not to produce a flash of light when a first test or sampling camera flash occurs, or is detected, and to produce a flash of light when a second camera flash is detected.

Preferably the bulb assembly further comprises a light source. The light source is preferably on the optical axis of the microscope, so as to illuminate the area of the retina visible through the eyepiece. Most preferably, light from the light source is focussed onto the flash generation means, so as to produce a virtual image of the light source in the same physical location as the flash generation means. This ensures that light generated by the slave flash travels to the location that is illuminated by the light source (and hence to the location which is visible through the eyepiece). The light from the bulb may be focussed using condensing optics or any other suitable means.

The synchronisation means preferably comprises a photo-detector.

Preferably the controller is arranged to distinguish between the first sampling flash and the second camera flash. Alternatively, the synchronisation means may be arranged to distinguish between the two flashes, and to send a signal to the controller after the detection of the main camera flash only. Alternatively the synchronisation means may be arranged only to detect the second main camera flash.

The bulb assembly is preferably removable, and is preferably replaceable (either after having been repaired, or with a similar unit). The slit lamp microscope is preferably the BM900, with its original bulb assembly having been removed and replaced with a bulb assembly according to the invention.

According to a second aspect of the invention we provide a bulb assembly for a slit lamp microscope, the bulb assembly comprising flash generation means able to produce a flash of light, and synchronisation means able to detect light from a camera flash, and a controller, the bulb assembly being arranged so that, in use, when the controller receives a signal from the synchronisation means indicating light from a camera flash has been detected, the controller causes the flash generation means to produce a slave flash of light.

The bulb assembly is preferably suitable for use in a prior art slit lamp microscope.

According to a third aspect of the invention we provide a method of taking a photograph of a subject through an eyepiece of a slit lamp microscope having a camera aligned with an eye piece of the microscope comprising the steps of:
operating a shutter release mechanism of the camera:
   generating at least one camera flash;
   detecting light from the camera flash;
   generating a slave flash of light in response to the camera flash, such that light from the slave flash travels along the optical axis of the microscope and illuminates the subject; and
   taking a photograph of the illuminated subject through the eyepiece of the microscope using the camera.

Preferably the subject comprises a patient's retina.

Preferably the slave flash is generated in a bulb assembly, the bulb assembly comprising flash generation means and synchronisation means. Most preferably the synchronisation means comprises a photo-detector.

According to a fourth aspect of the invention we provide a method for converting a prior art slit lamp microscope to a slit lamp microscope in accordance with the first aspect of the invention comprising the steps of:
removing an existing bulb assembly from the microscope; and
fitting the microscope with a bulb assembly according to the second aspect of the invention.

According to a fifth aspect of the invention we provide a slit lamp conversion kit, comprising a bulb assembly according to the second aspect of the invention, and a set of instructions to perform the method of the fourth aspect of the invention.

Preferably the kit also comprises a camera, and an adaptor for attaching the camera to an eyepiece of a slit lamp microscope. The kit may comprise a power supply adaptor, for connecting the bulb assembly to the power supply of a slit lamp microscope.

An embodiment of the invention will now be described in more detail, by way of example only, with reference to the following drawings in which:
**Figure 1** shows a perspective view of a prior art slit lamp microscope;
**Figure 1a** schematically shows the path of light through a slit lamp microscope;
**Figure 2** shows a digital camera and camera attachment;
**Figure 3** shows a digital camera attached to an eyepiece of a slit lamp microscope;
**Figure 4** shows a schematic view of a bulb assembly in accordance with the invention; and
**Figure 5** show a schematic view of a slit lamp microscope in accordance with the invention.

A slit lamp microscope 1 generally has a bulb assembly 3, optics 5, and eyepiece 7. In use, a patient (not shown) positions his or her chin on the chin rest 9, and his forehead against the head rest 10. The height of the chin and head rests are adjustable to suit the patient, so that an ophthalmologist using the microscope can position the patient's eye in line with the optical axis of the microscope. The bulb assembly 3 is a, 'top hat', approximately 3 inches (76 mm) in height and 3 inches (76mm) in diameter. The assembly 3 contains a bulb, the filament of which is intended to provide a continuous supply of light, which can be directed into the patient's eye by the ophthalmologist.

The term optical axis is used throughout this specification to mean the imaginary line that defines the path along which light propagates through the system. The exact path that the optical axis takes through the slit lamp microscope depends on the optics within the microscope itself, which are not described in detail within this specification. However, the optical axis of a slit lamp microscope is assumed to be arranged so that an object is visible through the eyepiece of the microscope when that object is on the optical axis of the system. The optical axis of a prior art slit lamp microscope is shown schematically in Figure 1a. It can be seen that light from a bulb 11 passes from the bulb assembly 3, through the microscope optics 5, and is directed by a mirror 12 into the patient's eye 14. The light is then reflected from the retina of the patient into the microscope eyepiece 7, allowing a clinician using the microscope to view the illuminated portion of the patient's retina. In practice, the ray of light reflected from the mirror 12 to the eye, and the ray of light reflected from the eye back to and through the mirror, will usually be on the same line/axis.

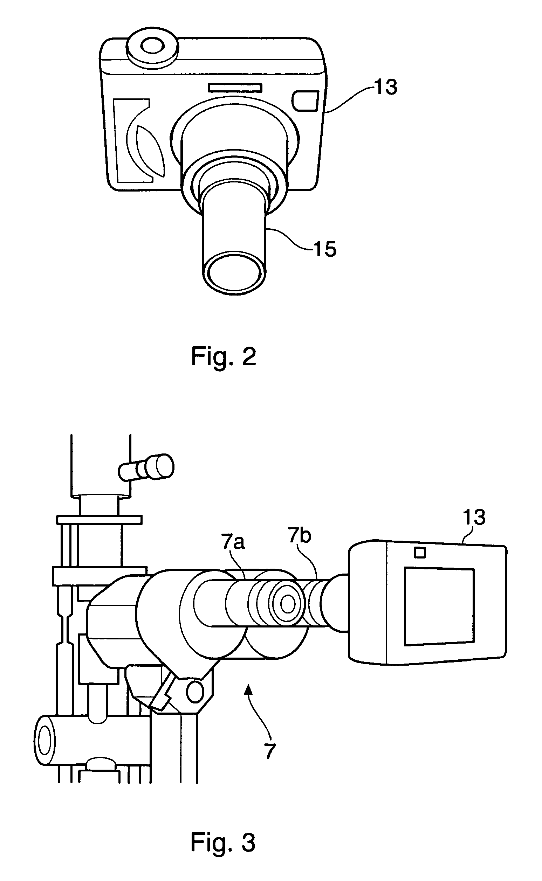

A photograph can be taken of the retina using such a prior art slit lamp by connecting an ordinary digital camera 13 to the eyepiece of the microscope using a camera attachment 15. Where the microscope is a binocular microscope, as shown in Figure 3, the camera can be attached to either one of the eyepieces 7a and 7b (in this case the camera is attached to eyepiece 7b). Although the cameras lens is in line with the optical axis of the eyepiece, the cameras flash is not. When a picture is taken with the digital camera little or no light from the camera's flash enters the patient's eye, as the microscope itself is in the way and the flash of the camera is not in the optical pathways of the slit lamp itself.

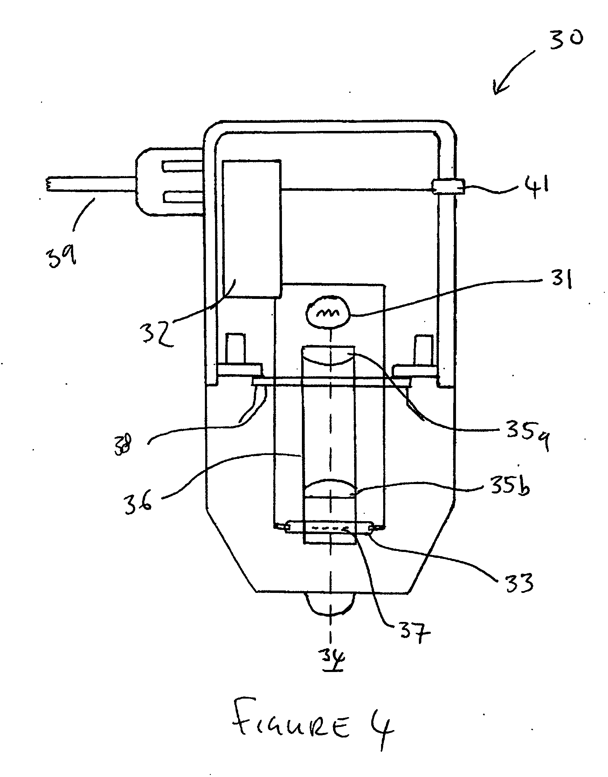

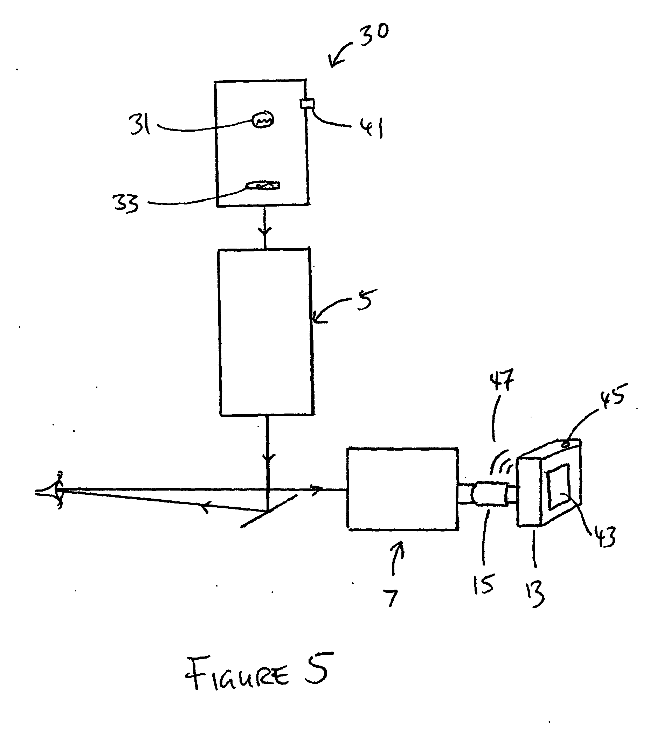

With reference to Figures 4 and 5, a slit lamp in accordance with the invention will now be described. The slit lamp has the same basic structure as the BM900 prior art slit lamp already described, except that the filament bulb assembly 3 has been replaced by a modified bulb assembly 30. The modified bulb assembly 30 is shown schematically in Figure 4.

Bulb assembly 30 contains a flash module 32 and a flash tube 33. Bulb assembly 30 is designed to be interchangeable with prior art bulb assembly 3, and so the flash tube 33 is positioned at the same relative position within the assembly 30 as the filament of the prior art light source 11 within the assembly 3. This ensures that light from the flash tube is directed and focussed by the optics 5 of the microscope along the optical axis of the microscope, in the same way as light from the filament of a prior art bulb assembly 3, and that the two bulb assemblies can be easily interchanged without needing to adjust the microscope optics. The assembly 30 has the same coupling formation (not shown) at its lower end as that of the conventional assembly 3.

A filament (or non-flash) light source 31 is also provided to illuminate the subject being viewed through the microscope, for example, when the clinician is examining the patient's eye, or when the clinician is preparing to take a photograph of the patient's eye. Light from the more-continuous filament light source also needs to be directed correctly by the microscope's optics 5. The light source 31 and the flash tube 33 are on the same axis, such that the light source 31 is also positioned on the optical axis 34 of the microscope. Condensing lenses 35a and 35b are provided, in a holder 36, which focus light from the light source 31 onto the flash tube 33, so as to create a virtual image 37 of the continuous light source 31 at the same physical location as the flash tube 33 (which location would have been occupied by the light source 11 in a prior art bulb assembly 3). Thus light from both the light source 31 and the flash tube 33 will be treated by the optics 5 (of the rest of the ophthalmoscope) in the same way. The background illumination from the light source 31 is formed via lenses onto the same position as the flash tube 33, which is in the exact same place as the original housing's tungsten filament. The flash tube 33 is transparent and relatively small, and these features help to avoid it causing a shadow on the image when the light source operates.

The continuous light source 31 is a miniature 6V halogen bulb or similar. Which is more compact than the original tungsten bulb provided in the existing known Haag Streit BM900 slit lamps. The bulb assembly 30 can be powered by the existing 6V power supply 39 to the bulb housing. All the components of the bulb assembly 30, including (but not limited to) the flash, an inverter to take 6V to 400V, the bulb, the circuitry inside the flash module, and the photo-detector, are powered using the same existing 6V supply.

From flange 38 downwards, we have positioned our components to replicate the original Haag tungsten bulb, and fit with the Haag Streit device. Flange 38 takes the place of the bulb plate used to locate the tungsten bulb in the prior art Haag BM900.

The bulb assembly 30 also has a photo-detector 41, the output of which is connected to the flash module 32. Flash module 32 contains electronic circuitry necessary to drive the transparent flash tube 33 in response to the output of the detector 41.

We have ensured that structure of the unit/assembly 30 is such that the optical components of the unit/assembly 30 are effectively positioned in exactly the same place in the optics of a Haag Streit BM900 as are the optical components of the original tungsten filament bulb assembly of the BM900. That is to say that the mounting structures and dimensions of our unit 30 cooperate with the structure of the BM900 that is left after the top of the BM900 is removed and the existing tungsten bulb assembly 3 is removed in such a way that the flash tube 33 is at the same point as the filament of the old, removed, tungsten bulb (to within 0.5mm), and that the background light source 31 is focussed onto the same point as that where the tungsten filament previously would have been. The need to match the spatial arrangement of the old tungsten bulb only really applies downwards, into the Haag Streit 900. There is no real need to be constrained extending upwards away from where the unit 30 is received in the external housing of the BM900 (which receives the unit 30 or the original tungsten bulb, as the case may be).

The operation of the bulb assembly in use will now be described with reference to Figure 5. Before taking a photograph, the clinician connects a camera 13 to the eyepiece 7 of the microscope using adaptor 15. The clinician lines up the microscope with the patient's retina, by looking in the screen 43 of the camera, or by looking through the eyepiece to which the camera is not attached. The light source 31 provides the clinician with the illumination necessary to achieve this. When the clinician has his desired subject in view he presses the shutter release button 45 on the camera in the normal way.

The camera then fires two flashes 47 in quick succession, a first sampling flash, and a second main flash. Both flashes are detected by photo-detector 41 and a signal indicating the detection of each flash is relayed to the flash module 32. The flash module 32 ignores the signal indicating detection of the first camera flash, as this is a sample flash, used by the camera to sense the required strength of flash required (which is then provided by the second main flash), and does not coincide with the camera shutter opening. Instead the flash module 32 drives the flash tube 33 when the detector 41 indicates that it has detected the second flash from the camera.

This method of awaiting a first sample flash, and firing a slave flash of the flash tube 33 on detection of a second main flash from the camera's own flash, ensures that the slave flash is fired at substantially the same time as, or very shortly after, the main flash, so that the subject in view is illuminated by the slave flash when the camera's shutter is open. A picture is thus taken of the illuminated area.

The camera's shutter speed can thus be considerably faster than when a bulb is connected to a prior art microscope without a flash. For example, the shutter speed may be increased from ¹/₂₀ of a second (in prior art devices, to obtain a light enough picture) to for example ¹/₁₀₀₀ of a second.

The bulb assembly 30 is removable, and may be connected above the optics 5 of the slit lamp microscope by any suitable means, such as thumbscrews. The bulb assembly is of course positioned so as to operate with the existing Haag Streit structure. This allows a clinician to purchase a bulb assembly 30, and unscrew the prior art assembly 3 from his existing ophthalmoscope (after detaching the power supply 39). He can then attach the new bulb assembly 30 to the microscope, and attach the existing power supply 39 to the new assembly. He is then ready to take photographs, by attaching a camera to the eyepiece of his microscope.

The bulb assembly 30 is provided with a connector (not shown) to which the power supply of a Haag BM900 can be attached. It is possible to connect a different or non-standard power supply (which is of the correct voltage, or which can be modulated to provide the correct voltage) by removing the existing connector from the power supply (perhaps by the simple method of cutting through the wiring) and replacing it (by rewiring if necessary) with a connector suitable for attachment to the connector of the module 30.

When a clinician has finished taking pictures he can easily remove the bulb assembly 30 and replace it with the original prior art bulb assembly 3. Alternatively, the clinician could leave the modified bulb assembly 30 in place, and use the modified slit lamp microscope as a simple viewing microscope. This can be done because the condensing optics 35 focus the light source 31 onto the site of the prior art light source 11, so allowing the bulb assembly to function well as a viewing slit lamp microscope if no camera is attached. Also the flash tube 33 is substantially optically transparent and so it does not obscure light from light source 31.

Alternatively, the modified slit lamp microscope could be used as a simple viewing microscope without removing the camera, as the camera's viewing screen can be used in place of the eyepiece of the microscope.

The bulb assembly 30 emulates the electrical contacts and dimensions of the original Haag Streit 900 tungsten bulb assembly so as to take up the same profile as the original bulb assembly (at least at the bottom end). The bulb assembly 30 is a fraction of the cost of a slit lamp microscope itself. It is also envisioned that the bulb assembly might be integral with a slit lamp microscope.

It will be appreciated that not all cameras may have a first sample flash. In such a case the flash module could be arranged to fire the flash on detection of a single flash. Rather than counting the number of flashes in order to decide when to fire the slave flash, the flash module could be arranged to detect differing properties of two camera flashes. For example, the first flash often comprises a higher proportion of infrared wavelengths, so as to be substantially invisible to the naked eye. The flash module could thus be arranged to only fire the slave flash on detection of a particular range of wavelengths in camera flash. Alternatively, the photo-detector could be arranged to only respond to the wavelength characteristics of the main flash, so that the sample flash need not be detected at all. There may be other characteristics which could be used to differentiate between a preliminary flash and a main flash that will be synchronised with actually taking a photograph.

The detector need not be a photo-detector, but could be arranged to detect any suitable signal from a camera, such as an audible or electronic signal.

It will be appreciated that the camera need not be connected to the slit lamp microscope by a camera adaptor, but could simply be held up to the eyepiece by the clinician. The clinician could thus focus the microscope onto the retina using both lenses of the binocular eyepiece, as he would during normal viewing, and then hold the camera up to one or the other of the lenses before pressing the shutter release button. However, this may introduce blurring into the picture due to movements of the clinician as the photograph is taken.

## Claims

1. A bulb assembly (30) for a slit lamp microscope (1) comprising flash generation means (33) able to produce a flash of light, synchronisation means (41) able to detect light (47) from a camera flash, and a controller (32), the bulb assembly being arranged so that, in use, when the controller (32) receives a signal from the synchronisation means (41) indicating light from a camera flash has been detected, the controller (32) causes the flash generation means (33) to produce a slave flash of light.

2. The bulb assembly of claim 1 wherein at least some of the light from the flash generation means (33) is directed along the optical axis of the microscope.

3. The bulb assembly of claim 1 or claim 2, further comprising a light source (31), wherein light from the light source is directed substantially along the optical axis of the microscope.

4. The bulb assembly of claim 3 wherein the light source (31) and the flash generation means (33) are located substantially on the optical axis of the microscope.

5. The bulb assembly of claim 3 or claim 4 wherein light from the light source (31) is focussed onto the flash generation means (33), so as to produce a virtual image of the light source in the same physical location as the flash generation means.

6. The bulb assembly of claim 5 wherein light from the light source is focussed onto the flash generation means using condensing lenses (35).

7. The bulb assembly of any one of claims 3 to 6 wherein the light source (31) is a halogen bulb.

8. The bulb assembly of any preceding claim wherein the controller (32) is arranged to cause the flash generation means (33) to produce a flash of light after receiving a signal indicating that a main camera flash has been detected by the synchronisation means (41).

9. The bulb assembly of claim 8 wherein the synchronisation means (41) and the controller (32) are arranged to cause the slave flash of light on detection of a main camera flash, and not to cause the slave flash of light in response to a preliminary, test, flash of light from the camera.

10. The bulb assembly of claim 8 wherein the synchronisation means (41) is arranged to send a signal to the controller (32) on detection of a main camera flash only.

11. The bulb assembly of claim 8 wherein the synchronisation means (41) is adapted to send a signal to the controller (32) on detection of both a sample flash and a main flash, the controller being arranged to distinguish between the sample and main camera flashes, and to cause the slave flash to occur only in response to the main flash.

12. The bulb assembly of any preceding claim wherein the synchronisation means (41) comprises a photo-detector.

13. A method of taking a photograph of a subject through an eyepiece (7) of a slit lamp microscope (1) having a camera (13) aligned with an eye piece of the microscope comprising the steps of:
operating a shutter release mechanism (45) of the camera:
generating at least one camera flash (47);
detecting light from the camera flash;
generating a slave flash of light in response to the camera flash, such that light from the slave flash travels along the optical axis of the microscope and illuminates the subject; and
taking a photograph of the illuminated subject through the eyepiece (7) of the microscope using the camera (13).

14. The method of claim 13 wherein the subject comprises a patient's retina.

15. A slit lamp microscope comprising an optical axis, flash generation means (33) able to generate a flash of light, synchronisation means (41) arranged to detect light from a camera flash, and a flash controller (32), the microscope being arranged so that, in use, when the flash controller (32) receives a signal from the synchronisation means (41) indicating that light (47) from a camera flash has been detected, the flash controller (32) causes the flash generation means (33) to produce a slave flash of light, such that at least some of the light from the slave flash travels along the optical axis of the microscope.

16. The microscope of claim 15 wherein the flash generation means (33), synchronisation means (41) and controller (32) are part of a self-contained bulb assembly (30).

17. The microscope of claim 16 wherein the bulb assembly is removable and replaceable.

18. A method for converting a prior art slit lamp microscope (1) to a slit lamp microscope in accordance with claim 15 comprising the steps of:
removing an existing bulb assembly (3) from the microscope; and
fitting the microscope with a bulb assembly (30) in accordance with any one of claims 1 to 14.

19. A slit lamp conversion kit, comprising a bulb assembly (30) in accordance with any one of claims 1 to 14, a camera (13), and an adaptor (15) for attaching the camera to an eyepiece (7) of a slit lamp microscope (1).
